# EUROPEAN PATENT APPLICATION

(11) **EP 4 534 068 A1**
(43) Date of publication of application: **09.04.2025**
(21) Application number: 24203639.0
(22) Date of filing: 30.09.2024
(51) Int. Cl.: A61K 8/02, A61K 8/04, A61K 8/19, A61K 8/34, A61K 8/9789, A61Q 5/02, A61K 8/73, A61K 8/81

(54) **DRY SHAMPOO SPRAY WITH LOW VOLATILE ORGANIC COMPOUND CONTENT**

(30) Priority: 03.10.2023 IT 202300020430
(71) Applicant: Cosmosol S.r.l., 20060 Mulazzano (LO) (IT)
(72) Inventor: Naso, Federica, 26813 Graffignana (IT); Racca, Stefania, 20098 San Giuliano Milanese (IT); Simonini, Stefano, 26837 Mulazzano (IT)
(74) Representative: De Anna, Pier Luigi

(57) **Abstract**

The present invention relates to a composition for dry shampoo and, in particular, a composition for non-inflammable spray dry shampoo, packaged using the aerosol technology with nitrogen propellant with a volatile organic compound content below 50%.

## Description

### FIELD OF THE INVENTION

The present invention relates to the field of cosmetic products and it relates to a composition for spray dry shampoo, with nitrogen as propellant.

### STATE OF THE ART

Dry shampoos are cosmetic products used for cleaning hair or, if need be, freshening the appearance thereof quickly and without the need for water.

Rather than referring to the final texture of the product, the expression dry shampoo refers to the fact that the product should be used "dry", that is it is applied directly on dry hair without having to use water after use.

As a matter of fact, applied on the scalp and on dry hair, these products carry out a sebum-adsorbing action capable of restoring the hair volume. Therefore, these products are popular among consumers due to their effectiveness and ease of removal, which occurs by simply brushing the hair and scalp.

Besides shampoos in powder form, dry shampoos in the form of spray, conditioned in pressurised cans which, exploiting the action of volatile organic compounds (VOCs in short), such as LPG, have the advantage, compared to powdered products, of being adapted to be vaporised on the hair in a fine and even manner, are known. Due to the presence of inflammable and pressurised gases, these products however reveal the drawback lying in the fact that they need to be moved and preserved away from heat sources.

Furthermore, it is known that besides contributing to atmospheric pollution, the use of volatile organic compounds also significantly contributes to "indoor" pollution. As a matter of fact, such compounds may cause to severe reactions, including eye, nose and throat irritation as well as headache. In addition, they have long-term effects, even the most severe, on the health of the user.

Spray dry shampoos generally consist of a functional substance, a solvent, a liquefied propellant and possible conditioners, preservatives, anti-caking agents and perfume. The active substances, mainly used, are rice starch, corn starch, modified starch and kaolin.

A further drawback of the dry shampoos available on the market lies in the fact that they often make the hair excessively opaque and the high percentage of adsorbent powder often results in unwanted white heat residue that is difficult to remove from the hair unless by washing.

In order to overcome this drawback, some manufacturers have provided for a wide range of dry shampoos that incorporate pigments adapted to different hair colours.

Furthermore, it is observed that the dry shampoos available on the market contain several synthetic components such as for example charging agents, which may be unwanted both from the point of view of the customer's perception and from the environmental standpoint.

EP3763350 describes a dry shampoo which has low residue on the hair and it is characterised by an aqueous formulation without natural starch. The formulation comprises insoluble powders and inorganic compounds such as silica, mica, iron oxides and other metals approved for cosmetic use, carbon, pvp.

WO2015/059170 describes a dry shampoo composition that is alcohol-free but characterised by a synthetic sebum-adsorbing agent.

WO 2020/127766 describes an aerosol device containing a hydro-alcoholic cosmetic composition in form of foam comprising an anionic surfactant, a powder and/or a fixative polymer, a non-ionic surfactant, a polysaccharide and an organic solvent.

The increasing awareness on the need for a responsible and sustainable behaviour, in order to limit global warming, and the evolution of the regulatory framework in this field have pushed the industry to commit to finding products adapted to ensure to the consumer a greater safety and lower environmental impact.

In order to reduce the VOC content in sprays, the state-of-the-art provides for using fluorinated propellants of synthetic origin such as propellant 152a (INCI: hydrofluorocarbon 152a). However, the replacement of volatile compounds of petrochemical origin with propellants of synthetic origin, does not solve the problems highlighted above.

As a matter of fact, most spray dry shampoos available on the market are products with a VOC percentage above 50% given that, besides propellants such as LPG, they use high percentages of alcohol as solvent for the adsorbent components. Therefore, there arises the need for novel compositions for dry shampoos to be used as an alternative to conventional products.

Finally, the use of nitrogen, a gas that is not harmful to the environment, as propellant for aerosol is known also in the cosmetic field. However, it should be observed that the use of nitrogen as propellant reveals technical problems when filling the aerosol product, mainly generated by the instability of the suspension. Furthermore, the use of a compressed gas such as nitrogen as propellant is considered inappropriate for cosmetic products containing powders, such as dry shampoos, given that it is not possible to guarantee the complete emptying of the content of the spray can. As a matter of fact, the force exerted by the compressed gas is not sufficient to expel all the product given that the powders, contained therein, tend to compact and deposit over time obstructing the dispenser channel and the valve of the can.

WO2022/013362 describes a haircare aerosol product, with a low volatile organic compound content, which uses a compressed gas as propellant and which, through a specific configuration of the valve, which uses a VPT flow regulator, enables to maintain the performance of the product, the durability and/or reduce the residual product to the minimum.

### DESCRIPTION OF THE INVENTION

An object of the present invention is to overcome the drawbacks of the prior art products with a spray dry shampoo composition that is inflammable, harmless to the environment and to the user, with a volatile organic compound level below 50% and free of fluorinated propellants.

Another object of the invention is to formulate a spray dry shampoo that does not use a volatile organic compound as propellant but which, overcoming the technical difficulties in this field, allows to guarantee a correct dispensing of the product and the complete emptying of the container.

A further object of the invention is to obtain a product that is highly adsorbent but which does not leave visible residues on the hair, irrespective of its colour, and therefore which does not need to incorporate additional pigments.

The invention attains the objects indicated above with a composition for dry shampoo which uses nitrogen as propellant, as shown in claim 1 of the patent, which is intended reported herein.

The composition for dry shampoo according to the invention preferably comprises:
- from 2% to 8% by weight of an adsorbing agent, preferably impalpable rice starch in powder form (INCI: Oryza sativa starch);
- from 30% to 60% by weight of a compound having the function of solvent/suspender for non-VOC powder, preferably deionised water (INCI: Water);
- from 30% to 50% by weight of a VOC solvent, preferably denatured ethyl alcohol (INCI: Denat. Alcohol);
- from 2% to 4% by weight of nitrogen (INCI: Nitrogen) as propellant.

It should be observed that the components of the compositions for dry shampoo according to the present invention are also indicated with reference to the International Nomenclature of Cosmetic Ingredients (INCI).

Furthermore, the invention provides for that the end product be conditioned in a can by correlating the percentage by weight of the components with the total volume of the final container.

Advantageously, the can is filled between 20% and 40%, more preferably between 25% and 35% and even more preferably between 28% and 32% by volume of the cosmetic composition and then pressurised with the nitrogen propellant for the remainder by volume.

Therefore, the cosmetic composition according to the invention, with low VOC content and with a percentage by volume of the product between 20% and 40% of the total volume of the can, advantageously, allows to increase the amount of cosmetic product carried to the hair and in the end product, increasing the durability thereof entirely without liquefied propellants and without the need of special valves with flow regulators.

Advantageously, the composition according to the invention may contain, additionally to the components listed above, from 0.2% to 2% by weight of a suspending agent, preferably a smectite clay in powder form (INCI: Hectorite) in order to improve the stability of the suspension in the filling production steps.

Other suspending agents in powder form which can be used in the composition are for example: Bentonite, Silica, Magnesium Aluminum Silicate, Disteardimonium Hectorite and the dispersions and mixtures thereof.

Therefore, the invention allows to overcome the technical problems of the prior art observed when filling the product with nitrogen propellant, due to the instability of the suspension, using a clay in powder form which, slowing the powder sedimentation time, has a suspension stabilising action.

Furthermore, advantageously, the presence of the suspending agent reduces the risk of inhomogeneity of the samples.

In order to improve the sprayability of the compound, besides the components indicated above, the composition according to the invention may contain an anionic surfactant which positively intervenes on the electrostatic charge of the adsorbing agent. The preferred anionic surfactant is Sodium laureth sulfate, present in the composition in percentage by weight comprised between 0.1% and 1%.

Other recommended but not preferred possible anionic surfactants are for example: alkyl glyceryl sulfates, acyl glutamates such as, by way of example, Sodium cocoyl glutamate, anionic glucosidic derivatives such as Disodium Laurylglucosides Hydroxypropyl Citrate; alkyl monoglyceride ether sulfates; alkyl monoglyceride sulfates; alkyl monoglyceride sulfonates; alkyl sulfonates; alkyl sulfosuccinates; alkyl ether sulfosuccinates; alkyl sulfosuccinamates; alkyl amido sulfosuccinates; alkyl carboxylates; alkyl 5 amido ether carboxylates; alkyl succinates; fatty acyl sarcosinates; fatty acyl amino acids; fatty acyl taurates; fatty alkyl sulphur acetates; and mixtures thereof.

In a preferred formulation, the adsorbing agent of the composition consists of impalpable rice starch in powder form (INCI: Oryza sativa starch). However, other recommended but not preferred possible adsorbent agents, with reference to the INCI classification, may be selected from: Tapioca starch, PVP, Zea Mays (Corn) Starch, Aluminium Starch Octenylsuccinate.

The denatured alcohol used in the preferred composition according to the invention, such as VOC solvent, is preferably denatured alcohol of the type defined under letter d) of article 2 paragraph 2 of the Italian Ministerial Decree dated 9 July 1996, that is obtained by adding, to each hectolitre of anhydrous alcohol of:
- natural or synthetic moss: grams 39.5;
- tertbutyl alcohol (TBA): grams 78.8.

Other recommended but not preferred possible alcohols include all the other types of alcohol approved by the regulations in force in the cosmetic field.

The present invention allows to attain the further advantage of avoiding the compaction of the powders and the resulting clogging of the dispensing channel and of the valve of the can, given that the cleaning of the channel and valve can be guaranteed due to the incorporation of water in the composition, besides the fact of enabling to use a smaller amount of adsorbent powder with respect to the known formulae.

The composition for dry shampoo of the present invention may also be used to clean hair of any colour, therefore also dark hair, given that there are no unappealing light coloured residues on the hair after use. This is believed to be guaranteed by the fineness, lightness and resistance of the composition to caking. Therefore, advantageously, unlike the case in some prior art formulations, there is no need for, incorporating a pigment in the composition so as to adapt it to use on dark hair.

The product with nitrogen according to the invention is characterised by a formula which contains a low percentage of alcohol (lower than 50% by weight), with the advantage of reducing discomfort at the level of the scalp which could be caused by the prior art formulations with greater percentages.

Furthermore, the presence of water, which acts as a powder suspending agent and replaces conventional volatile organic compounds, allows to add cosmetic ingredients such as, by way of example, glycerine or glycerine extracts or probiotics, with emollient, anti-reddening, soothing activity.

Furthermore, it can be observed that, advantageously, the product subject of the invention lasts longer, considering the same volume of the can in which it is conditioned, with respect to conventional products which use LPG as propellant. The filling ratio of the end product may range from 20:80 (base:propellant) to 40:60 (base:propellant) by volume. Preferably, the 30:70 ratio enables to obtain a greater duration of the product compared to conventional dry shampoos while maintaining the final price of the product competitive compared to the standard.

The duration is approximately greater than 30% compared to a prior art spray dry shampoo. As a matter of fact, by using nitrogen, the base/gas by weight ratio moves from 10-20/90-80 of a dry shampoo with LPG as propellant to 99-95/1-5 by using the formulation according to the invention with nitrogen, with resulting in positive impact on the duration of the product.

Furthermore, through a flammability test conducted by an independent laboratory (in compliance with Directive 75/324/EEC et seq. and regulation 1272/2008 and subsequent updates), it was observed that the products resulting from the composition subject of the invention are entirely non-inflammable, an important and advantageous characteristic both for the supply chain and for the end consumer.

Lastly, in the possible embodiments, the composition for dry shampoo according to the invention may incorporate substantially or exclusively natural ingredients, without any preclusions in this sense.

### EMBODIMENTS

Some embodiments of the invention, provided by way of nonlimiting example, are reported below.

The table below reports four formulation examples of the present invention, indicating the percentages by weight of the components:

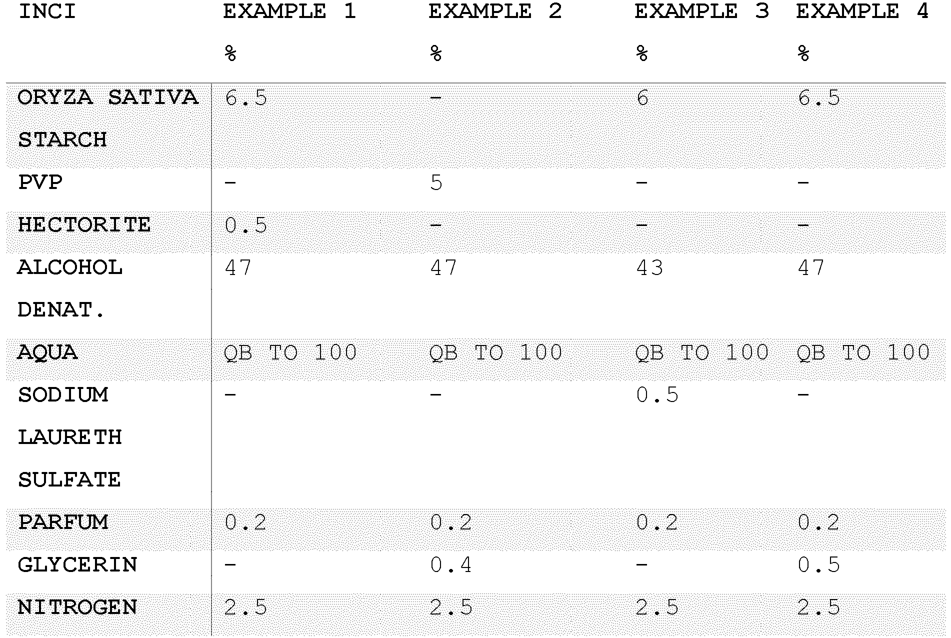

All the formulations provided by way of example comprise, as solvent, a mixture of deionised water and denatured ethyl alcohol to obtain a value by weight of VOC on the end product below 50.

Furthermore, all compositions of the present invention use, as propellant, nitrogen, present in the examples in a percentage of 2.5%. However, such value may clearly range from 1% to 10% depending on the ingredients used.

In the preferred formulations, the adsorbing agent consists of impalpable rice starch in powder form (INCI: Oryza sativa starch). However, other appropriate adsorbent agents may be used, such as for example PVP, indicated in the example 2, or, generally as Tapioca starch, Zea Mays (Corn) Starch, Aluminium Starch Octenylsuccinate.

In example 1, in order to improve the stability of the suspension in the filling production steps and reduce the risk of inhomogeneity of the samples, the composition contains 0.5% by weight of a suspending agent, a smectite clay in powder form (INCI: Hectorite).

As mentioned above, it is clear that smectite clay may also be replaced by another suspending agent in powder form, for example Bentonite, Silica, Magnesium Aluminum Silicate, Disteardimonium Hectorite and the dispersions and mixtures thereof.

As indicated in the formulation of example 3, the composition may contain an anionic surfactant, generally from 0.1% to 1% by weight, so as to improve the sprayability of the compound, positively interfering with the electrostatic charge of the adsorbing agent.

The preferred anionic surfactant is Sodium laureth sulfate. However, other possible recommended but not preferred anionic surfactants have been listed above.

As shown in the examples, further ingredients may be added to the composition of the present invention, without altering the characteristics thereof, including:
fragrances, essential oils, plant extracts, antimicrobial agents, preservative system, dyes, skin conditioners, humectants, perlators, opacifiers, mica, exfoliants, lipids, vitamins, solar filters, polishing agents, silicones, esters, extracts, moisturising agents for the scalp and/or for the hair and mixtures thereof.

The formulations outlined by way of example allow to provide compositions for spray dry shampoos that are fine, light, highly adsorbent and with low volatile organic compound content.

As a matter of fact, the compositions advantageously free of propellants conventionally used for conditioning spray cans such as LPG (INCI: Butane, Propane; Isobutane), DME (INCI: Dimethylether), 152a (INCI: Hydrofluorocarbon 152a), Solstice Propellant HFO1234ze (INCI: Tetrafluoropropene), Solstice Enhance HFC-134a (INCI: Chlorotrifluoropropene) and mixtures thereof.

Besides allowing the packaging of the dry shampoo in pressurised spray cans with an eco-friendly propellant such as nitrogen and having a VOC content below 50%, the compositions for dry shampoo according to the invention are advantageously adapted to clean even dark hair, without the need to incorporate additional dyes.

Given that the composition according to the invention may incorporate substantially or exclusively natural ingredients, without any preclusions in this sense, it is therefore possible to obtain a product characterised by a high natural index, comprised between 95 and 100, a value which indicates in which percentage a cosmetic product consists of elements of natural origin according to the ISO 16128 guidelines. Such result clearly cannot be obtained by using conventional formulae and propellants.

## Claims

1. Spray dry shampoo with a volatile organic compound content not exceeding 50% **characterized in that** it comprises:
- from 2% to 8% by weight of an adsorbing agent in powder form;
- from 30% to 60% by weight of a non-volatile organic compound, preferably water, having the function of solvent/suspender for powder;
- from 30% to 50% by weight of a VOC solvent;
- from 1% to 10% by weight of nitrogen as propellant;
and wherein the percentage for filling the cosmetic product into the can, before pressurisation, is comprised between 20% and 40% of the total volume of the can.

2. Spray dry shampoo according to claim 1, **characterised in that** said nitrogen as propellant is present between 2% and 4% by weight of the composition.

3. Spray dry shampoo according to the preceding claim, **characterised in that** the compound having the function of solvent/suspender for powder not belonging to volatile organic compounds is comprised between 40% and 45% by weight of the composition.

4. Spray dry shampoo according to one of the preceding claims, **characterised in that** the VOC solvent is denatured ethyl alcohol for cosmetic use.

5. Spray dry shampoo according to the preceding claim, **characterised in that** the VOC solvent is comprised between 42% and 48% by weight of the composition.

6. Spray dry shampoo according to one of the preceding claims **characterised in that** the percentage for filling the cosmetic product is present by volume between 25% and 35% and more preferably between 28% and 32% on the pressurised finished product.

7. Spray dry shampoo according to one of the preceding claims, **characterised in that** the adsorbing agent in powder form is Oryza sativa starch, or alternatively, Tapioca starch, PVP, Zea Mays (Corn) Starch, Aluminium Starch Octenylsuccinate.

8. Spray dry shampoo according to the preceding claim, **characterised in that** said adsorbing agent is present in percentages by weight comprised between 5% and 7%.

9. Spray dry shampoo according to one of the preceding claims, **characterised in that** it contains a suspending agent, in percentages by weight comprised between 0.2% and 1% of the composition, preferably Hectorite, or alternatively, Bentonite, Silica, Magnesium Aluminum Silicate, Disteardimonium Hectorite and dispersions and mixtures thereof.

10. Spray dry shampoo according to one of the preceding claims, **characterised in that** it contains an anionic surfactant, in percentages by weight comprised between 0.2% and 1%.

11. Spray dry shampoo according to the preceding claim, **characterised in that** said anionic surfactant, is Sodium laureth sulfate or, alternatively, alkyl glyceryl sulfates, acyl glutamate sulfates of alkyl ether monoglycerides, alkyl monoglyceride sulfonates, alkyl sulphonates, alkyl sulphosuccinates, alkyl ether sulphosuccinates, alkyl amido sulfosuccinates, alkyl carboxylates, alkyl 5-amido ether carboxylates, alkyl succinates, fatty acyl sarcosinates, fatty acyl amino acids, fatty acyl taurates, fatty alkyl sulphur acetates, and mixtures thereof.

12. Spray dry shampoo according to one of the preceding claims, **characterised in that** it comprises at least one preservative, in a total amount not exceeding 2% by weight of the composition.

13. Spray dry shampoo according to one of the preceding claims, **characterised in that** it comprises at least one fragrance, in a total amount not exceeding 1% by weight of the composition.
